# EUROPÄISCHE PATENTSCHRIFT

(11) **EP 2 771 245 B1**
(45) Veröffentlichungstag und Bekanntmachung des Hinweises auf die Patenterteilung: **25.05.2016**
(21) Anmeldenummer: 12770073.0
(22) Anmeldetag: 27.09.2012
(51) Int. Cl.: B65C 9/00, B67C 3/22, B65B 59/04, B65B 59/02, B29C 49/78, B29C 49/42, B29C 31/00, B23Q 3/155

(54) **BEHÄLTERBEHANDLUNGSANLAGE UND HANDHABUNGSVORRICHTUNG**
CONTAINER TREATMENT SYSTEM AND HANDLING DEVICE
INSTALLATION DE TRAITEMENT DE CONTENANTS ET DISPOSITIF DE MANIPULATION

(30) Priorität: 28.10.2011 DE 102011054890
(43) Veröffentlichungstag der Anmeldung: 03.09.2014
(60) Teilanmeldung: 16157922.2
(73) Patentinhaber: Krones Aktiengesellschaft, 93073 Neutraubling (DE)
(72) Erfinder: WINZINGER, Frank, 93073 Neutraubling (DE)
(74) Vertreter: Benninger, Johannes
(86) Internationale Anmeldenummer: PCT/EP2012/069054
(87) Internationale Veröffentlichungsnummer: WO 2013/060549

(56) Entgegenhaltungen:
- EP-A1- 0 572 107
- EP-A1- 0 849 060
- EP-A2- 2 199 220
- EP-A2- 2 292 402
- EP-A2- 2 306 255
- DE-U1- 20 000 417
- JP-A- 8 001 709

## Beschreibung

Die vorliegende Erfindung betrifft eine Behälterbehandlungsanlage und eine Handhabungsvorrichtung.

Große Abfüll- und / oder Verpackungsanlagen umfassen in der Regel eine Mehrzahl von Behälterbehandlungsmodulen, beispielsweise Module für das Herstellen von Behältern oder Flaschen, für das Befüllen, Verschließen, Etikettieren etc. In Abhängigkeit vom herzustellenden Produkt müssen die einzelnen Module umgerüstet werden. Insbesondere müssen bestimmte Arbeitswerkzeuge ausgetauscht und / oder Formatteile an das neue Produkt angepasst werden. Soll beispielsweise von Produkt Nr.1 auf Produkt Nr.2 umgestellt werden, kann es notwendig sein, in einem Blasformmodul die Blasformen auszutauschen, damit eine neue Behälterform produziert werden kann. Weiterhin müssen beispielsweise bestimmte Formatteile im nachgeordneten Füller etc. an die neue Behälterform angepasst werden.

Zum Austausch von Arbeitswerkzeugen und / oder zum Anpassen von Formatteilen an einzelnen Behälterbehandlungsmodulen sind jeweils spezielle Wechselvorrichtungen an jedem Modul vorgesehen, die die entsprechenden auszutauschenden Arbeitswerkzeuge oder Werkzeuge zur Anpassung der Formatteile bereitstellen. Diese Wechselvorrichtungen werden je nach Produkt mit den entsprechenden Arbeitswerkzeugen oder Werkzeugen bestückt. In Abhängigkeit davon, welche Arbeitswerkzeuge gewechselt oder welche Formatteile angepasst bzw. verstellt werden müssen, können auch mehrere Wechselvorrichtungen pro Modul benötigt werden. Diese Wechselvorrichtungen werden jeweils nur zum Umrüsten eines bestimmten Arbeitswerkzeugs und / oder zum Anpassen eines bestimmten Formatteils bei einem Produktwechsel benötigt. Somit stehen diese Wechselvorrichtungen die meiste Zeit ungenutzt im Weg und nehmen Platz in Anspruch, da sie die Gesamtgröße der Anlage erhöhen. Der Benutzer benötigt für jedes Modul ein oder mehrere dieser Wechselvorrichtungen, was sich in hohen Anschaffungskosten für die Module niederschlägt.

DE 4242925 A1 beschreibt eine Transporteinrichtung für Flaschenbehandlungsmaschinen, bei der zum Wechseln von Arbeitswerkzeugen eine Greifeinrichtung mit Greifkopf vorgesehen ist. Die Greifeinrichtung ist am Maschinengestell angebracht und über eine Steuereinrichtung gesteuert.

EP 0985633 A1 zeigt eine Vorrichtung zum automatischen Wechsel von Füllköpfen. Hierbei ist an der seitlichen Peripherie der Behälterbehandlungsmaschine ein in unterschiedliche Positionen verfahrbarer Bestückungs- und Wechselautomat vorgesehen, mit welchem Bauteile an den Behandlungsköpfen angebracht, vorhandene Bauteile von diesen entfernt und in eine Vorratsposition bringbar und andere Bauteile aus ihrer Vorratsposition holbar und mit den Behandlungsköpfen verbindbar sind.

EP2292402 offenbart eine Magazinvorrichtung (Handhabungsvorrichtung) mit einer Vielzahl von Aufnahmemitteln um einzelne Blasformen getrennt voneinander aufzunehmen. Die Magazinvorrichtung kann zum Wechseln von Blasformen einer Blasmaschine (Behälterbehandlungsanlage) an eine Blasmaschine zum automatischen Entnehmen der Blasformen angedockt werden.

Aufgabe der Erfindung ist, eine Behälterbehandlungsanlage zur Verfügung zu stellen, bei welcher Arbeitswerkzeuge einfach und effektiv ausgetauscht bzw. Formatteile einfach und effektiv angepasst werden können und wobei gleichzeitig eine bessere Auslastung der notwendigen Wechselvorrichtungen erfolgt.

Die obige Aufgabe wird durch eine Behälterbehandlungsanlage und eine Handhabungsvorrichtung gelöst, die die Merkmale in den Patentansprüchen 1 und 11 umfassen. Weitere vorteilhafte Ausgestaltungen werden durch die Unteransprüche beschrieben.

Die Erfindung bezieht sich auf eine Behälterbehandlungsanlage zur Herstellung und / oder Bearbeitung von Produkten und / oder Produktgruppen. Die Erfindung betrifft weiterhin eine Handhabungsvorrichtung zum Austauschen von Arbeitswerkzeugen und / oder zur Anpassung von Formatteilen an einer Behälterbehandlungsanlage.

Insbesondere bezieht sich die Erfindung auf eine Behälterbehandlungsanlage zur Herstellung von Behältern, Befüllung dieser Behälter mit einem vorzugsweise flüssigen Inhalt, Verschließen der befüllten Behälter, Etikettieren der Behälter und / oder Zusammenstellen und Verpacken der Behälter in Gruppen. Eine solche Behälterbehandlungsanlage umfasst in der Regel mindestens zwei Behälterbehandlungsmodule. Beispielsweise handelt es sich hierbei um eine Flaschenherstellungs-, -befüllungs- und / oder -verpackungsanlage, die unter anderem eine Heizstrecke, ein Blasformmodul, ein Etikettiermodul und einen Füller umfasst. Weitere Module werden in den Ausführungsbeispielen beschrieben. Unter Behälterbehandlungsanlage ist aber auch eine Anlage aus mehreren Produktionslinien zu verstehen, wobei jede Produktionslinie jeweils aus mindestens zwei Modulen besteht. Dabei können die Produktionslinien jeweils dieselbe Modulzusammenstellung aufweisen. Es ist aber auch denkbar, dass die mindestens zwei Produktionslinien jeweils unterschiedliche Behälterbehandlungsmodule umfassen.

Die Behälterbehandlungsanlage umfasst mindestens zwei Arbeitsbereiche, in denen bei einem Produktwechsel Arbeitswerkzeuge ausgetauscht und / oder Formatteile angepasst werden müssen. Beispielsweise müssen bei einem Produktwechsel die Blasformen in einem Arbeitsmodul ausgetauscht werden und die Führungsgeländer an einem Transportmittel, über das die blasgeformten und / oder schon gefüllten Flaschen weiteren Verarbeitungsvorrichtungen zugeführt werden, an die jeweilige Breite der Flaschen angepasst werden, in dem die Führungsgeländer zueinander verstellt werden.

Die Behälterbehandlungsanlage umfasst mindestens eine zu den mindestens zwei Arbeitsbereichen bewegbare, insbesondere verfahrbare, Handhabungsvorrichtung. Die Handhabungsvorrichtung ist über Zentriermittel selektiv mit den Arbeitsbereichen der Behälterbehandlungsanlage verbindbar. Die Handhabungsvorrichtung ist eine so genannte Wechselvorrichtung und dient dem Austausch des mindestens einen Arbeitswerkzeugs und / oder der Anpassung des Formatteils. Hierfür umfasst die Handhabungsvorrichtung entsprechende Mittel.

Jeder der Arbeitsbereiche der Behälterbehandlungsanlage umfasst jeweils eine erste Verbindungvorrichtung mit Zentriermittel. Die Handhabungsvorrichtung weist dagegen mindestens eine zweite Verbindungsvorrichtung mit Zentriermittel auf. Die mindestens eine zweite Verbindungsvorrichtung mit Zentriermittel der Handhabungsvorrichtung ist korrespondierend zu den jeweils ersten Verbindungsvorrichtungen mit Zentriermitteln der mindestens zwei Arbeitsbereiche der Behälterbehandlungsanlage ausgebildet. Über die zweite Verbindungsvorrichtung mit Zentriermittel und eine der ersten Verbindungsvorrichtungen kann eine form- und / oder kraftschlüssige Verbindung zwischen einem der Arbeitsbereiche und der Handhabungsvorrichtung hergestellt werden. Insbesondere bedeutet dies, dass die mobile Handhabungsvorrichtung entweder mit dem einen der zwei Arbeitsbereiche oder mit dem anderen der beiden Arbeitsbereiche verbunden werden kann. Die Handhabungsvorrichtung ist also nicht einem der beiden Arbeitsbereiche fest zugeordnet. Insbesondere wird die Handhabungsvorrichtung nur im Wechselmodus, d.h. wenn eine Behälterbehandlungsanlage für einen Produktwechsel umgerüstet werden muss, am entsprechenden Arbeitsbereich mit der Behälterbehandlungsanlage bzw. dem Behälterbehandlungsmodul verbunden. Befindet sich das Behälterbehandlungsmodul im Produktionsbetrieb, ist die Handhabungsvorrichtung passiv bzw. kann an einem anderen Arbeitsbereich der Behälterbehandlungsanlage verwendet werden. Es wäre auch denkbar, die Handhabungsvorrichtung mit einer in der Produktion mithelfenden Funktion auszurüsten, wenn die Handhabungsvorrichtung gerade nicht für einen Formatwechsel benötigt bzw. eingesetzt wird. Die Handhabungsvorrichtung kann dann beispielsweise mit einem Puffer für Flaschen oder mit einem Sammelvolumen für aus der Produktion ausgeschleuste Flaschen ausgestattet werden.

Die Arbeitsbereiche können Teil der Behälterbehandlungsanlage bzw. des Behälterbehandlungsmoduls sein. Alternativ sind die Arbeitsbereiche nicht Bestandteil der Behälterbehandlungsanlage bzw. des Behälterbehandlungsmoduls, sondern stellen jeweils entsprechend zugeordnete Andockstellen am Hallenboden der Halle dar, in der sich die Behälterbehandlungsanlage befindet.

Bei den Verbindungsvorrichtungen mit Zentriermitteln an der Behälterbehandlungsanlage handelt es sich beispielsweise um so genannte Zentrierstifte oder Zentrierkonen. Die Handhabungsvorrichtung weist entsprechende Aufnahmen auf, in die die Zentrierstifte oder Zentrierkonen greifen. Dabei wird beispielsweise eine Rastverbindung, Steckverbindung o.ä. zwischen den Zentriermitteln hergestellt. Dies bewirkt eine gerichtete form- und / oder kraftschlüssige Verbindung zwischen der Handhabungsvorrichtung und der Behälterbehandlungsanlage. Die Handhabungsvorrichtung wird über die Zentriermittel direkt für den Wechsel- bzw. Anpassungsprozess ausgerichtet. Ebenso kann es sich bei den Verbindungsvorrichtungen mit Zentriermitteln lediglich um Mittel zum reinen Informationsaustausch handeln. Hierunter fallen beispielsweise eine optische Erkennung und Selbstausrichtung der Handhabungsvorrichtung zu den Modulen oder eine Erkennung eines Andockens der Handhabungsvorrichtung seitens des Moduls, beispielsweise über an der Handhabungsvorrichtung angeordnete Transponder. Auch kann es sich bei der Verbindung um einen Passstift, welcher in eine Bohrung einführbar ist, um eine Anschlagfläche oder eine Bodenmulde handeln. Weitere dem Fachmann bekannte Zentriermittel sollen ebenfalls umfasst sein.

Der erste Arbeitsbereich ist einem ersten Modul der Behälterbehandlungsanlage, beispielsweise einem Blasformmodul, und der zweite Arbeitsbereich ist einem zweiten Modul der Behälterbehandlungsanlage, beispielsweise einem Füller, zugeordnet. Die Handhabungsvorrichtung kann somit entweder am Blasformmodul oder am Füller eingesetzt werden.

Die Handhabungsvorrichtung kann mindestens zwei unterschiedliche Verbindungsvorrichtungen zum Ankuppeln an unterschiedliche Arbeitsbereiche umfassen. Gemäß einer bevorzugten Ausführungsform kann jedoch vorgesehen sein, dass die Handhabungsvorrichtung eine so genannte Universal- Verbindungsvorrichtung umfasst und dass die Arbeitsplätze jeweils korrespondierende Universal-Verbindungsvorrichtungen umfassen.

Die Handhabungsvorrichtung umfasst vorzugsweise mindestens ein Magazin für Arbeitswerkzeuge, das jeweils mit unterschiedlichen Arbeitswerkzeugen bestückt werden kann. Alternativ oder zusätzlich kann die Handhabungsvorrichtung Werkzeuge zum Austausch der Arbeitswerkzeuge und / oder zur Anpassung von Formatteilen umfassen, wobei die Werkzeuge ebenfalls ausgetauscht werden können. Das Magazin kann als hängende oder stehende Lagerung für die Arbeitswerkzeuge und / oder Werkzeuge ausgeführt sein. Beispielsweise wird die Handhabungsvorrichtung benötigt, um Blasformen an einem Blasformmodul auszutauschen. Dies ist immer nur bei einem Produktwechsel notwendig, so dass die Handhabungsvorrichtung die meiste Zeit nicht benötigt wird. Die Handhabungsvorrichtung kann in den Stillstandzeiten, d.h. in den Zeiten, in denen kein Wechsel von Arbeitswerkzeugen oder kein Anpassen von Formatteilen an der ersten Arbeitsposition erfolgt, von dem Bediener der Anlage oder automatisiert umgerüstet werden, d.h. mit neuen Arbeitswerkzeugen und / oder Werkzeugen bestückt und ggf. umprogrammiert werden. Danach kann die Handhabungsvorrichtung an der zweiten Arbeitsposition oder einer weiteren Arbeitsposition eingesetzt werden. Beispielsweise werden die nicht mehr benötigten Blasformen in ein Lager gebracht und stattdessen Füllventile im Magazin angeordnet, so dass die Handhabungsvorrichtung nunmehr verwendet werden kann, um beispielsweise einen Füller für ein neues Produkt vorzubereiten. Die Handhabungsvorrichtung kann aber auch mehrere Magazine für unterschiedliche Garnituren bzw. Wechselteile aufweisen. Die Handhabungsvorrichtung kann auch wiederum mindestens eine eigene Andockstation aufweisen, an welcher andere Wechseleinrichtungen andocken können, um beispielsweise ganze Wechselmagazine austauschen zu können.

Die Handhabungsvorrichtung kann zum einen die notwendigen Arbeitswerkzeuge und / oder Werkzeuge zum Umrüsten der Behälterbehandlungsanlage bereitstellen. Weiterhin kann die Handhabungsvorrichtung vorzugsweise die Mechanik und / oder Elektronik bereitstellen, um die Umrüstung der Behälterbehandlungsanlage in der jeweiligen Arbeitsposition weitgehend selbständig, insbesondere halb- oder vollautomatisch vorzunehmen. Insbesondere können über die Handhabungsvorrichtungen auch Updates der Betriebssoftware an die Module übertragen werden, z.B. wenn die Behälterbehandlungsanlage auf die Produktion neuer Flaschen, welche zuvor nicht verarbeitet wurden, umgestellt werden soll.

Die Handhabungsvorrichtung kann beispielsweise mindestens einen Austauschstern umfassen, der Greifvorrichtungen zum Greifen der entsprechenden Arbeitswerkzeuge aufweist. In einer weiteren Ausführungsform handelt es sich bei der Handhabungsvorrichtung um einen Roboter, welcher eine Mehrzahl von über Achsen bewegbare Arme und / oder Greifwerkzeuge aufweist, um die entsprechenden Umrüstarbeiten vorzunehmen. Im einfachsten Fall handelt es sich beispielsweise um einen Automaten mit pneumatischen Stellantrieben, wie er bekannterweise in Blasformmodulen eingesetzt wird. Die jeweiligen Antriebe von Behandlungsmodul und Handhabungsvorrichtung sind insbesondere synchronisierbar. Bevorzugt handelt es sich um Servomotoren, es ist aber auch eine Synchronisation über eine Getriebekupplung möglich.

Alle auszuwechselnden Arbeitswerkzeuge sind bevorzugt über einen schnell zu öffnenden bzw. zu verschließenden Arretiermechanismus, beispielsweise über einen Rastmechanismus, an den Behälterbehandlungsmodulen angebracht, so dass die Arbeitswerkzeuge werkzeugfrei auf Knopfdruck o.ä. austauschbar sind. Alternativ kann die Handhabungsvorrichtung aber auch entsprechende Werkzeuge, beispielsweise Schraubwerkzeug o.ä., zum Austausch der Arbeitswerkzeuge umfassen.

Neben einem Wechsel von Arbeitswerkzeugen kann die Handhabungsvorrichtung auch Einstellarbeiten an den Behälterbehandlungsmodulen vornehmen, beispielsweise die Anpassung von Kurvenbahnen zur Steuerung bestimmter Behälterbehandlungsmodule.

Vorzugsweise umfasst die Handhabungsvorrichtung Transportmittel. Diese sind notwendig, um die Handhabungsvorrichtung schnell an ihren jeweiligen Einsatzort an den unterschiedlichen Arbeitspositionen bringen zu können. Im einfachsten Fall weist die Handhabungsvorrichtung Räder auf und wird von einem Bediener manuell zur jeweiligen Arbeitsposition geschoben. Alternativ kann die Handhabungsvorrichtung mit einem eigenen Antrieb ausgestattet sein. Beispielsweise kann die Handhabungsvorrichtung zudem über ein eigenes optisches Erkennungssystem verfügen, dass der Handhabungsvorrichtung ermöglicht, die richtige Position an der Maschine selbst zu suchen und einzunehmen. Gemäß einer Ausführungsform ist vorgesehen, dass die Handhabungsvorrichtung über ein Schienensystem bewegt wird. Die Schienen erstrecken sich dabei zumindest zwischen der ersten Arbeitsposition und der zweiten Arbeitsposition an der Behälterbehandlungsanlage. In diesem Fall kann die Position der Handhabungsvorrichtung über eine Steuerungsvorrichtung vorgegeben werden. Die Handhabungsvorrichtung wird dann automatisch über das Schienensystem zur richtigen Arbeitsposition geleitet.

Gemäß einer Ausführungsform umfasst die Handhabungsvorrichtung eine Steuerungsvorrichtung, damit das Umrüsten der Behälterbehandlungsanlage automatisch gesteuert erfolgen kann. Insbesondere steuert die Steuerungsvorrichtung den Austausch der Arbeitswerkzeuge an der jeweiligen Arbeitsposition und / oder die Anpassung der Formatteile an der jeweiligen Arbeitsposition und / oder die korrekte Positionierung der Handhabungsvorrichtung an der jeweiligen Arbeitsposition.

Die Steuerungsvorrichtung wird entsprechend der neuen Produktvorgaben programmiert und die Handhabungsvorrichtung mit den notwendigen Arbeitswerkzeugen und / oder Werkzeugen bestückt, so dass nach dem Ankuppeln an ein Behälterbehandlungsmodul die Umrüstarbeiten mit Hilfe der Handhabungsvorrichtung manuell, halb- oder vollautomatisch vorgenommen werden. D.h. entweder umfasst die Handhabungsvorrichtung alle notwendigen Mittel, um automatisch die notwendigen Umrüstarbeiten vorzunehmen, oder aber die Handhabungsvorrichtung stellt notwendige Mittel bereit, damit ein Bediener die notwendigen Umrüstarbeiten manuell oder halbautomatisiert durchführen kann.

Beispielsweise errechnet die Steuerungsvorrichtung aufgrund der neuen Produktvorgaben, welche Arbeitswerkzeuge auszuwechseln oder welche Formatteile anzupassen sind. Die Ergebnisse dieser Berechnung werden dem Bediener der Behälterbehandlungsanlage beispielsweise über eine Anzeigeeinheit dargestellt. Die Handhabungsvorrichtung kann aber auch die benötigten Arbeitswerkzeuge und / oder Werkzeuge beispielsweise automatisiert aus einem Lager entnehmen, diese in einem eigenen internen Magazin anordnen, automatisch die korrekte Arbeitsposition an der Behälterbehandlungsanlage einnehmen, über die Verbindungsvorrichtung form- und / oder kraftschlüssig an die Behälterbehandlungsanlage ankuppeln und den entsprechenden Austausch der Arbeitswerkzeuge oder die entsprechende Anpassung der Formatteile vornehmen. Die Steuerung der Handhabungsvorrichtung kann auch die zeitgünstigste Reihenfolge errechnen, in der der Formatwechsel durchgeführt werden kann. Diese können den Mitarbeitern über die Anzeige mitgeteilt werden. Ebenso sind insbesondere die nötigen Informationen über die Arbeitssicherheit in der Steuerung hinterlegt, so dass von Mitarbeitern gleichzeitig durchzuführende Arbeiten nicht von dem Einsatz der Handhabungsvorrichtung behindert oder gefährdet werden können.

Weiterhin kann die Handhabungsvorrichtung Schnittstellen zur Versorgung der Handhabungsvorrichtung mit Energie, Druckluft und / oder Informationen verfügen. Beim Ankuppeln der Handhabungsvorrichtung an das jeweilige Behälterbehandlungsmodul werden diese Verbindungen manuell, halb- oder vollautomatisch hergestellt.

Gemäß einer weiteren Ausführungsform wird die Handhabungsvorrichtung an einer der Arbeitspositionen mit der Behälterbehandlungsanlage verbunden und insbesondere an eine Steuerung des Behälterbehandlungsmoduls oder der Behälterbehandlungsanlage angekoppelt. Bei dieser Ausführungsform erhält die Handhabungsvorrichtung alle notwendigen Information zu den auszutauschenden Arbeitswerkzeugen und / oder anzupassenden Formatteilen über die Modulsteuerung des Behälterbehandlungsmoduls oder der Behälterbehandlungsanlage.

Die Handhabungsvorrichtung kann sensorische Mittel zur Erkennung von Arbeitswerkzeugen und / oder Werkzeugen umfassen. Über den Sensor kann die Handhabungsvorrichtung beispielsweise die im internen Magazin gespeicherten Arbeitswerkzeuge erkennen und die richtigen Arbeitswerkzeuge zum Einbau in das jeweilige Behälterbehandlungsmodul auswählen. Alternativ kann die Handhabungsvorrichtung sensorische Mittel umfassen, die die richtigen Arbeitswerkzeuge in einem externen Lager identifizieren. Insbesondere sind die Werkzeuge oder Garnituren, aber auch die korrespondierenden Stellen der Module mit RFID-Chips, Barcodes oder anderen Codierungen versehen, die eine der Arbeitswerkzeuge Erkennung erleichtern.

Gemäß einer Ausführungsform kann die Handhabungsvorrichtung ein Magazin für sämtliche Typen eines Arbeitswerkzeugs eines Behälterbehandlungsmoduls umfassen. Beispielsweise kann die Handhabungsvorrichtung also verschiedene Blasformen für ein Blasformmodul aufnehmen. Das Magazin kann die Arbeitswerkzeuge (Blasformen) in Typ- Gruppen in Untermagazinen lagern und je nach Programm den gewünschten Arbeitswerkzeugs- Typ (Blasform- Typ) abrufen. Die richtigen Arbeitswerkzeuge (Blasformen) werden anhand des Programms identifiziert, aus dem internen Magazin entnommen und verbaut. Hierbei kann vorgesehen sein, dass jedem Arbeitswerkzeug-Typ ein bestimmter Platz im Magazin zugewiesen ist, so dass die richtigen Arbeitswerkzeuge aufgrund ihrer Positionierung im Magazin entnommen werden. Alternativ kann ein Detektionssystem dafür vorgesehen sein. Beispielsweise können die Arbeitswerkzeuge jeweils mit einem RFID- Chip o.ä. ausgestattet sein, dass eine einfache Identifizierung und Zuordnung erlaubt.

Gemäß einer weiteren Ausführungsform kann die Handhabungsvorrichtung auch nur die Mechanik zur Verfügung stellen, um Arbeitswerkzeuge auszuwechseln, welche in einem externen Magazin an dem Behälterbehandlungsmodul oder anderweitig gelagert sind. Hierzu ist die Handhabungsvorrichtung insbesondere mit einem Detektionssystem ausgestattet, das die unterschiedlichen Arbeitswerkzeuge in dem externen Lager erkennt, sich anhand des Programms den richtigen Arbeitswerkzeugs - Typ auswählt und an der entsprechenden Stelle in das Behälterbehandlungsmodul einbaut.

Gemäß einer weiteren Ausführungsform enthält die Handhabungsvorrichtung ein Magazin für auszuwechselnde Arbeitswerkzeuge, in dem mehr Austauschteile vorhanden sind, als eigentlich zum Umrüsten des Behälterbehandlungsmoduls benötigt werden. Die Position eines Arbeitswerkzeugs in der Handhabungsvorrichtung und am Behälterbehandlungsmodul wird von einer Steuerungsvorrichtung, beispielsweise der internen Steuerung der Handhabungsvorrichtung oder der Modulsteuerung der Behälterbehandlungsanlage gespeichert, so dass die Handhabungsvorrichtung die Arbeitswerkzeuge gezielt aus dem Magazin entnimmt und so einsetzt, dass die Austauschteile gleichmäßig abgenutzt werden. Dadurch wird gewährleistet, dass ein weitgehend gleichmäßiger Verschleiß der Arbeitswerkzeuge erfolgt. Weiterhin kann vorgesehen sein, dass die Handhabungsvorrichtung einen Sensor zur Erkennung fehlerhafter Arbeitswerkzeuge und / oder Werkzeuge umfasst. Wenn der Sensor fehlerhafte Arbeitswerkzeuge und / oder Werkzeuge erkennt, gibt er über eine Anzeigevorrichtung ein Signal aus, damit der Bediener das fehlerhafte Arbeitswerkzeug und / oder Werkzeug erkennt und dieses ersetzt oder repariert. In einem automatisierten System kann vorgesehen sein, dass die Handhabungsvorrichtung das fehlerhafte Arbeitswerkzeug und / oder Werkzeug automatisch aussondert und mit einem neuen Arbeitswerkzeug und / oder Werkzeug aus einem Lager ersetzt.

Beim Umrüsten wird das Behälterbehandlungsmodul vorzugsweise im Wechselzyklus und insbesondere taktweise betrieben. Insbesondere umfasst der Arbeitsbereich einen Wechselbereich. Beispielsweise wird pro Takt eine im Wechselbereich angeordnete Blasform entnommen und in das interne Magazin der Handhabungsvorrichtung oder in ein externes Magazin eingelagert. Anschließend wird das Blasrad weiter gedreht, so dass sich nunmehr die benachbarte alte Blasform im Wechselbereich befindet. Nunmehr wird diese ausgetauscht. Dieser Prozess wird fortgesetzt, bis alle alten Blasformen aus dem Blasformmodul entfernt worden sind. In einem weiteren Zyklus werden taktweise die neuen Blasformen im Blasformmodul angebracht. Alternativ wird pro Takt ein Formatteil entsprechend dem neuen Produkt angepasst. Weiterhin kann vorgesehen sein, dass der Wechsel der Arbeitswerkzeuge anstelle von taktweise auch kontinuierlich durchgeführt wird. Benötigen die Arbeitswerkzeuge noch eine Vorbereitung, so kann diese auch von dem jeweiligen Modul zur Verfügung gestellt werden. Bei einem Blasrad kann beispielsweise eine für einen Wechselbetrieb zustellbare Kurve vorhanden sein, durch welche Blasformträger, in denen die zu wechselnden Blasformen angeordnet sind, schon kurz vor dem Wechsel entriegelt und geöffnet werden können. Auf diese Art und Weise muss die Handhabungsvorrichtung nicht alles selbst bewerkstelligen, so dass auch dadurch Zeit eingespart werden kann. Nach dem Wechsel der Blasform würde dann das Schließen derselben durch eine zweite zustellbare Kurve erfolgen. Die Funktion kann auch mit einer zustellbaren Doppelkurve erfolgen.

Gemäß einer alternativen Ausführungsform kann die Handhabungsvorrichtung auch beide Schritte, d.h. Entnehmen des alten Arbeitswerkzeugs und Anbringen des neuen Arbeitswerkzeugs simultan in einem Zyklus durchführen. Insbesondere umfasst die Handhabungsvorrichtung in diesem Fall mindestens ein Leer- Magazin für die Aufnahme der entnommenen Arbeitswerkzeuge und mindestens ein mit anzubringenden Arbeitswerkzeugen befüllbares Speicher- Magazin, dass vorher manuell oder automatisiert mit den entsprechenden benötigten Arbeitswerkzeugen entsprechend dem umzurüstenden Behälterbehandlungsmodul befüllt worden ist. Dieser Prozess kann auch kontinuierlich durchgeführt werden. Es wäre es sogar möglich, den Prozess während der laufenden Produktion durchzuführen. Hierzu könnte man den Wechselautomaten zwischen den Einlauf und den Auslauf einer Behandlungsmaschine setzen, d.h. insbesondere in einen Bereich in der Produktion, in dem keine Behälter behandelt werden.

Bei einem Austausch von Arbeitswerkzeugen oder beim Anpassen von Formatteilen ist es meist notwendig, dass sich Maschinenteile des jeweiligen Behälterbehandlungsmoduls bewegen, um die Arbeitswerkzeuge oder Formteile jeweils in die richtige Wechselposition zu bringen. Dabei muss auf die Sicherheit der Bediener geachtet werden. Insbesondere muss verhindert werden, dass diese mit sich bewegenden, beispielsweise rotierenden, Maschinenteilen in Berührung kommen. Um die Sicherheit der Bediener zu gewährleisten, werden beispielsweise in einem Wechselzyklus die Sternräder der zu wechselnden jeweiligen Arbeitswerkzeuge unabhängig von gerade nicht zu wechselnden Sternrädern angetrieben. Weiterhin kann aus Sicherheitsgründen vorgesehen sein, dass die Handhabungsvorrichtung derart mit der Arbeitsposition verbunden wird, dass kein Bediener mehr beim Wechselvorgang dazwischengreifen kann.

Weiterhin wird eine Handhabungsvorrichtung zum Austauschen von Arbeitswerkzeugen und / oder zur Anpassung von Formatteilen an mindestens zwei unterschiedlichen Arbeitsbereichen einer Behälterbehandlungsanlage, die mobil an jeweils einem von mindestens zwei Arbeitsbereichen an der Behälterbehandlungsanlage eingesetzt werden kann, veröffentlicht.
Insbesondere weist die Handhabungsvorrichtung die bereits oben beschriebenen Charakteristika auf. Die Handhabungsvorrichtung, die dem Austausch von Arbeitswerkzeugen und / oder dem Anpassen von Formatteilen einer Behälterbehandlungsanlage bei einem Produktwechsel dient, ist somit an mindestens zwei Stellen bzw. Arbeitsbereichen an der Behälterbehandlungsanlage einsetzbar. Vorzugsweise kann die Handhabungsvorrichtung universell an verschiedenen Behälterbehandlungsmodulen einer Produktionslinie oder an mehreren in einer Halle angeordneten Produktionslinien verwendet werden. Dadurch ist es nicht mehr notwendig, für jedes auszutauschende Arbeitswerkzeug bzw. für jedes anzupassende Formatteil jedes Behälterbehandlungsmoduls eine eigene Wechselvorrichtung bereitzustellen, die die meiste Zeit nicht benutzt wird. Durch die mehrfache Nutzung wird die Handhabungsvorrichtung wesentlich besser ausgelastet. Für den Benutzer spiegelt sich dies in geringeren Kosten wieder, da er nicht für jedes Modul eine eigene Wechselvorrichtung mitkaufen muss.

Während die Handhabungsvorrichtung(en) den Wechselprozess durchführt(en), ist der Bediener der Anlage bzw. Anlagen frei, sich um weitere Umrüstungen zu kümmern, die nicht von den Handhabungsvorrichtungen vorgenommen werden bzw. werden können.

Eine solche Handhabungsvorrichtung wird vorzugsweise an einer Behälterbehandlungsanlage bzw. einem Behälterbehandlungsmodul eingesetzt, dass eine Endlosförderung umfasst, insbesondere Rundläufermodule wie Blasformmodule, Füller etc. oder Module mit einem umlaufenden Kettenantrieb, wie beispielsweise Linear-Heizöfen etc.

Die Erfindung betrifft weiterhin eine Handhabungsvorrichtung zum Austauschen von Arbeitswerkzeugen und / oder zum Anpassen von Formatteilen einer Behälterbehandlungsanlage zur Herstellung von Produkten und / oder Produktgruppen, nach Anspruch 11.
Die Handhabungsvorrichtung umfasst eine Verbindungsvorrichtung mit Zentriermittel und wird einem Wechselbereich der Behälterbehandlungsanlage zugeordnet. Der Wechselbereich der Behälterbehandlungsanlage weist eine korrespondierende Verbindungvorrichtung mit Zentriermittel auf. Über die Verbindung der beiden Verbindungsvorrichtungen wird eine form- und / oder kraftschlüssige Verbindung zwischen dem Wechselbereich und der Handhabungsvorrichtung hergestellt. Alternativ kann die Handhabungsvorrichtung auch ortsfest an der Behälterbehandlungsanlage angebracht sein. Die Handhabungsvorrichtung umfasst mindestens ein Leer- Magazin für die Aufnahme der zu entnehmenden Arbeitswerkzeuge und mindestens ein Speicher-Magazin. Das Speicher- Magazin wird jeweils entsprechend dem herzustellenden oder zu bearbeitenden Produkt mit Arbeitswerkzeugen und ggf. notwendigen Werkzeugen befüllt. Die Entnahme der alten Arbeitswerkzeuge und das Anbringen der neuen Arbeitswerkzeuge kann mit dieser Handhabungsvorrichtung zeitgleich durchgeführt werden. D.h. während ein altes Arbeitswerkzeug an einer ersten definierten Position im Behälterbehandlungsmodul entnommen wird, wird ein neues Arbeitswerkzeug an einer zweiten Position eingesetzt. Vorzugsweise erfolgt die Entnahme des alten Arbeitswerkzeugs und das Anbringen des neuen Arbeitswerkzeugs jeweils um eine oder nur wenige Position(en) versetzt.

Ein solcher Wechsel findet somit beispielsweise an einem Blasformmodul folgendermaßen statt: Zuerst wird eine alte Blasform an einer ersten Position entnommen und im Leer- Magazin angeordnet. Anschließend dreht das Blasrad um eine Position weiter. Während nunmehr die alte Blasform an der zweiten Position entnommen wird, wird gleichzeitig eine neue Blasform an der ersten Position befestigt. Anschließend dreht das Blasrad wiederum um eine Position weiter. Nun wird die alte Blasform an der dritten Position entnommen und eine neue Blasform an der zweiten Position befestigt usw.

Gemäß einer bevorzugten Ausführungsform werden die Magazine kontinuierlich, insbesondere rotativ, angetrieben und die Arbeitswerkzeuge kontinuierlich ausgewechselt. Entweder erfolgt der Wechsel der Arbeitswerkzeuge durch die Handhabungsvorrichtung mit verringerter Geschwindigkeit im Vergleich zur normalen Produktionsgeschwindigkeit der Behälterbehandlungsvorrichtung. Im Idealfall kann ein Wechsel in Produktionsgeschwindigkeit durchgeführt werden. Gemäß einer weiteren Ausführungsform ist vorgesehen, die Produktion während des Wechselns nicht zu unterbrechen. Hierzu kann entweder ein Behälter auf dem Zuförderer oder vorher ausgeschleust werden. Oder aber der Behälter wird gar nicht durch die Behälterbehandlungsvorrichtung produziert. Somit wird verhindert, dass in der durch den Entnahme des ersten Arbeitswerkzeugs zwischenzeitlich entstehenden Lücke ein Behälter den Wechsel stört. Gemäß einer weiteren Ausführungsform kann auch vorgesehen sein, das Arbeitswerkzeug inklusive dem zuletzt produzierten oder behandelten Behälter zu entnehmen. Der Behälter muss anschließend durch die Handhabungsvorrichtung ausgeschleust und beispielsweise verworfen werden.

Alternativ oder zusätzlich zur zeitgleichen simultanen Entnahme bzw. dem Einsetzen kann die Entnahme der alten Arbeitswerkzeuge und das Anbringen der neuen Arbeitswerkzeuge mit dieser Handhabungsvorrichtung auch kontinuierlich durchgeführt werden. Beispielsweise wird bei dieser Alternative zuerst das erste Magazin komplett mit den nicht mehr benötigen Blasformen befüllt und erst anschließend werden die neuen Blasformen in einem zweiten Umlauf der Maschine in die Maschine vom Vorratsmagazin übergeben und montiert. Bei der Kombination der simultanen und kontinuierlichen Entnahme bzw. dem Einsetzen wird das erste Magazin mit den alten Werkzeugen befüllt, bis die entstehende Lücke beim zweiten Magazin angekommen ist, wobei dieses in diese erste Lücke das erste neue Werkzeug einsetzt. Ab diesem Zeitpunkt beginnt für den Rest der auszuwechselnden Werkzeuge ein kontinuierlicher Lauf, wobei sich sowohl das Behandlungskarussell als auch die beiden Magazinvorrichtungen kontinuierlich weiterdrehen, bis schließlich jedes alte Werkzeug von jeder Station des Behandlungskarussells entnommen wurde und das erste Magazin keine Werkzeuge mehr aufnehmen muss. Das zweite Magazin arbeitet noch so lang weiter, bis alle Lücken auf dem Behandlungskarussell mit Werkzeugen besetzt sind. Mit Ausnahme des Beginns und des Endes des Austausch- Zyklus erfolgt somit ein simultaner und kontinuierlicher Austausch der Werkzeuge.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.
Figur 1 zeigt eine schematische Seitenansicht einer erfindungsgemäßen Handhabungsvorrichtung neben einer Verarbeitungsvorrichtung.
Figur 2 zeigt die Verwendung einer erfindungsgemäßen Handhabungsvorrichtung an einer aus Transportsternen bestehenden Übergabevorrichtung.
Figur 3 zeigt die Verwendung einer erfindungsgemäßen Handhabungsvorrichtung an einem Etikettiermodul.
Figur 4 zeigt die Verwendung einer erfindungsgemäßen Handhabungsvorrichtung an einer Blasmaschine.
Figur 5 zeigt die Verwendung einer erfindungsgemäßen Handhabungsvorrichtung an einem Füller.
Figur 6 zeigt die Verwendung einer erfindungsgemäßen Handhabungsvorrichtung in einer Abfüllanlage.
Figur 7 und Figur 8 zeigen eine weitere Ausführungsform zur Verwendung einer erfindungsgemäßen Handhabungsvorrichtung an einem Füller.
Figur 9 zeigt eine weitere Ausführungsform einer erfindungsgemäßen Handhabungsvorrichtung.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie die erfindungsgemäße Vorrichtung ausgestaltet sein kann und stellen keine abschließende Begrenzung dar.

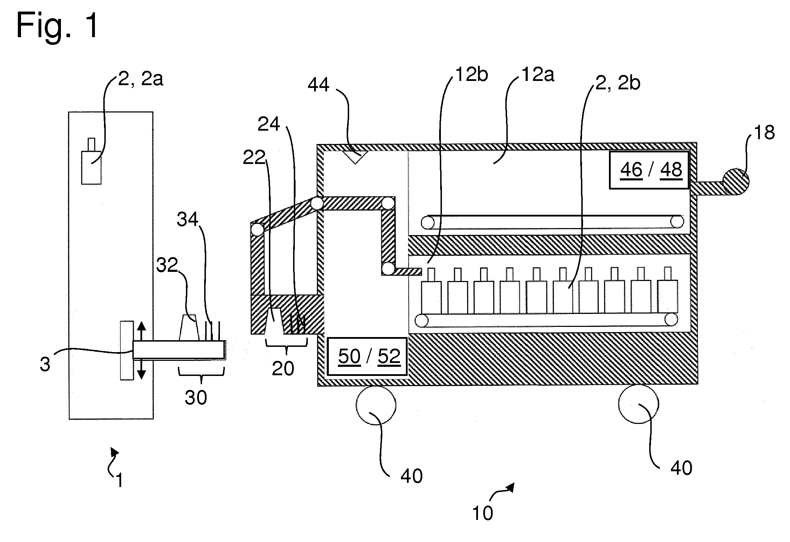

**Figur 1** zeigt eine schematische Seitenansicht eines Wechselautomaten 10 neben einer Verarbeitungsvorrichtung 1. Im vorliegenden Fall müssen bei einem Produktwechsel die Arbeitswerkzeuge 2 produktabhängig ausgetauscht werden. Beispielsweise müssen die in der Verarbeitungsvorrichtung 1 befindlichen Arbeitswerkzeuge 2a gegen die Arbeitswerkzeuge 2b ausgetauscht werden. Der Wechselautomat 10 umfasst mindestens zwei Magazine 12a, 12b zur Aufnahme von Arbeitswerkzeugen 2, 2a, 2b.

Der Wechselautomat 10 umfasst mindestens eine Schnittstelle 20 und die Verarbeitungsvorrichtung 1 umfasst mindestens eine korrespondierende Verbindungsvorrichtung 30. Die Schnittstelle 20 und die Verbindungsvorrichtung 30 weisen jeweils mechanische Verbindungs- und / oder Zentierelemente auf. Die Verbindungsvorrichtung 30 weist beispielsweise mindestens einen Zentrierstift oder Zentrierkonus 32 auf, der in eine korrespondierende Zentrieröffnung 22 der Schnittstelle 20 greift. Somit kann der Wechselautomat 10 gerichtet an der Verarbeitungsvorrichtung 1 angebracht werden, so dass der Wechselautomat 10 gleich korrekt arbeiten kann.

Weiterhin umfasst die Schnittstelle 20 elektrische und/oder pneumatische Verbindungselemente 24 zur Verbindung mit korrespondierenden elektrischen und/oder pneumatischen Verbindungselementen 34 der Verbindungsvorrichtung 30. Über die Schnittstelle 20 und die Verbindungsvorrichtung 30 können somit weitere Verbindungen zwischen dem Wechselautomat 10 und der Verarbeitungsvorrichtung 1 hergestellt werden, beispielsweise kann der Wechselautomat 10 dadurch an das Stromnetz der Verarbeitungsvorrichtung 1, ein Steuerungsgerät der Verarbeitungsvorrichtung 1, an das Druckluftsystem der Verarbeitungsvorrichtung 1 etc. angeschlossen werden.

Der Wechselautomat 10 weist Räder 40 auf, so dass er entweder über einen eigenen Antrieb (nicht dargestellt) oder von Hand verfahren werden kann. Hierfür ist beispielsweise ein Schiebegriff 18 vorgesehen. Der Wechselautomat 10 ist weiterhin entweder mit einer Batterie 50 und einem eigenen Antrieb 52 für weitere notwendige Funktionen versehen. Weiterhin kann vorgesehen sein, dass der Wechselautomat 10 über entsprechende Schnittstellen jeweils selbst mit Energie, Druckluft und / oder Informationen versorgbar ist.

Falls dies notwendig ist, kann vorgesehen sein, dass der an der Verarbeitungsvorrichtung 1 angekoppelte Wechselautomat 10 beispielsweise über einen Hebemechanismus 3 an der Verarbeitungsvorrichtung 1 angehoben und in die korrekte Position verfahren wird.

Vorzugsweise wird die Verarbeitungsvorrichtung 1 im Wechselzyklus taktweise betrieben, so dass pro Takt ein Arbeitswerkzeug 2, 2a entnommen werden kann. Das Arbeitswerkzeug 2, 2a wird anschließend in das Magazin 12a des Wechselautomaten 10 eingeordnet. In einem weiteren Zyklus werden die neuen Arbeitswerkzeuge 2, 2b wiederum taktweise an entsprechender Stelle in bzw. an der Verarbeitungsvorrichtung 1 angebracht.

Im Wechselautomat 10 ist ein Sensor 44 zum Erkennen fehlerhafter Arbeitswerkzeuge 2, 2a, 2b und / oder der Identifikation bzw. auch des Formats der Arbeitswerkzeuge 2, 2a, 2b in den Magazinen 12a, 12b vorgesehen. Detektiert der Sensor 44 ein fehlerhaftes Arbeitswerkzeug 2, 2a, 2b, gibt er eine entsprechende Fehlermeldung aus, so dass das fehlerhafter Arbeitswerkzeuge 2, 2a, 2b entsorgt und ersetzt oder repariert wird. Die Ausgabe der Fehlermeldung erfolgt beispielsweise über eine Anzeigetafel 46, die beispielsweise Teil einer Steuerungseinheit 48 ist.

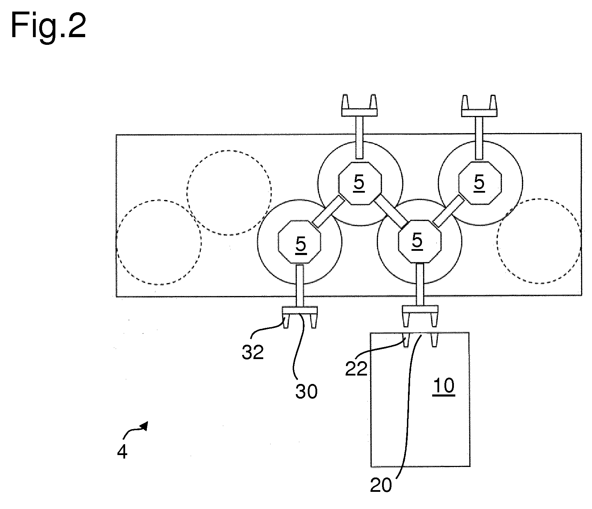

**Figur 2** zeigt die Verwendung eines Wechselautomaten 10 an einer aus Monotec- Transfersternen 5 bestehenden Übergabevorrichtung 4. Eine solche Übergabevorrichtung 4 wird beispielsweise verwendet, um einzelne Transfersterne 5 variabel an Schnittstellen - diese sind hier achteckig dargestellt - miteinander durch nicht gekennzeichnete Streben verbinden zu können und u.U. die zu reinigenden Außenflächen eines Füllbereichs möglichst gering zu halten. In Abhängigkeit vom Produkt, müssen über die Monotec- Transfersterne 5 verschiedene Behälter transportiert werden, so dass bei einem Produktwechsel insbesondere die Greiferklammern für die Behälter (nicht dargestellt) ausgetauscht werden müssen. Die Monotec- Transfersterne 5 weisen jeweils eine Verbindungsvorrichtung 30 mit Zentrierbolzen 32 auf, über die der Wechselautomat 10 an den jeweiligen Monotec- Transferstern 5 andocken kann, um die Greiferklammern (nicht dargestellt) auszutauschen. Die Verbindungsvorrichtung 30 kann vorteilhafterweise ebenfalls an den Schnittstellen in gleicher Weise wie die Streben angebracht werden.

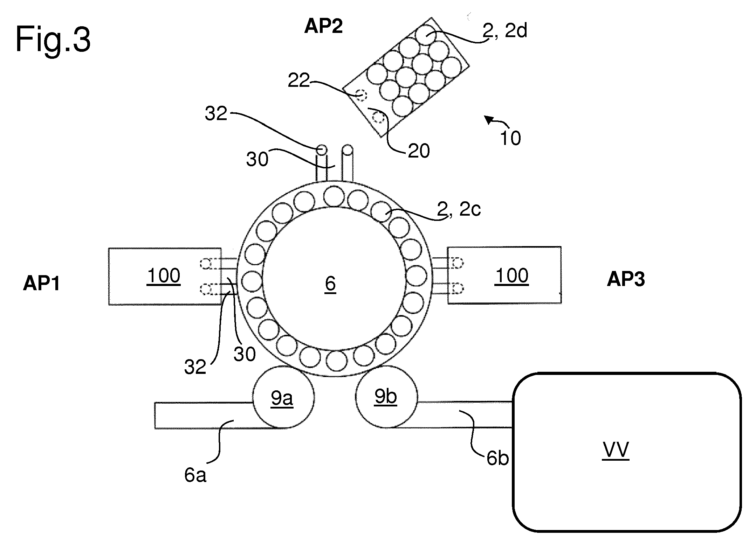

**Figur 3** zeigt die Verwendung eines Wechselautomaten 10 an einem Etikettiermodul 6. Die zu etikettierenden Flaschen (nicht dargestellt) werden über einen Zuförderer 6a und einen Einlaufstern 9a zum Etikettiermodul 6 transportiert und über einen Abförderer 6b und einen Auslaufstern 9b weiteren Verarbeitungsvorrichtungen VV zugeführt.

Bei einem Produktwechsel müssen bei einem Etikettiermodul 6 eine Reihe von unterschiedlichen Arbeitswerkzeugen 2 ausgetauscht und / oder angepasst werden. Dazu zählen beispielsweise die Zentrierglocken zum Festspannen der Flaschen auf den Drehtellern; die Drehteller zum Drehen der Flaschen bei der Etikettierung; die Dorne zum Aufweiten von schlauchförmigen Etiketten; die Stäbe und / oder deren zugehörige Führungen bzw. Stellantriebe zum Dehnen der Etiketten und Überziehen der Flaschen mit dem Etikett bei der Etikettierung mit dehnbaren schlauchförmigen Etiketten; die Palletten zum Transportieren und Beleimen von Etiketten von einem Entnahmemagazin zu einer Abgabe; Leimbehälter inkl. Leim; die Leimschaber zum Abschaben von Leim an der Leimwalze; die Bürsten zur Etikettenanbürstung an die Flasche; die Greiferzylinder zum Aufbringen von Etiketten an der vorbeikommenden Flasche; die Vakuumzylinder zum Transport und Halten von Etiketten bei der Etikettierung von zu schneidendem Bandetikettenmaterial; Einteilschnecken; Führungsschienen für Flaschen; Dorne, Klammern, Halterungen zum Transport von Flaschen, Steuerungen für Drehteller (mechanisch oder elektrisch) etc. So müssen in einer Ausführungsform, in der die Etikettiermaschine zwischen Blasmaschine und Füller angeordnet ist, beispielsweise auch die Bodenteller, welche zur Abstützung der noch vom Blasprozess heißen Flasche dienen, wobei die Flasche mit Druckluft zum Etikettieren vorgespannt wird, ausgewechselt werden. Weiterhin kann es notwendig sein, die Etiketten selbst auszutauschen und entsprechende Magazine für Einzeletiketten, Rollen mit Endlosetiketten - beispielsweise Sleeve oder Rundumetikett - bereitzustellen oder Druckaggregate zum direkten Bedrucken von Etiketten oder Flaschen auszutauschen oder bereitzustellen, dazu gehören zudem entsprechende Druckköpfe, Einstellungen für solche, Druckfarbenkanister etc.

Bei dem Etikettiermodul 6 selbst könnten beispielsweise die bereits vorhandenen Zentrierelemente 32 für die Etikettieraggregate 100 verwendet werden, um den Wechselautomaten 10 über eine korrespondierende Schnittstelle 20 ankuppeln zu lassen. Insbesondere kann vorgesehen sein, dass mehrere Wechselautomaten 10 anstelle der Etikettieraggregate 100 gleichzeitig an jeweils unterschiedlichen Arbeitspositionen AP1, AP2, AP3 an dem Etikettiermodul 6 ankuppeln, um simultan mehrere Arbeitswerkzeuge 2, 2c, 2d auszutauschen und / oder anzupassen. Wenn ein Formatwechsel an den Etikettieraggregaten 100 selbst durchzuführen ist, beispielsweise ein Wechsel von Paletten, Leimwalzen etc., können diese auch mit einer entsprechenden Verbindungsvorrichtung bzw. Zentrierelementen ausgestattet sein (nicht dargestellt).

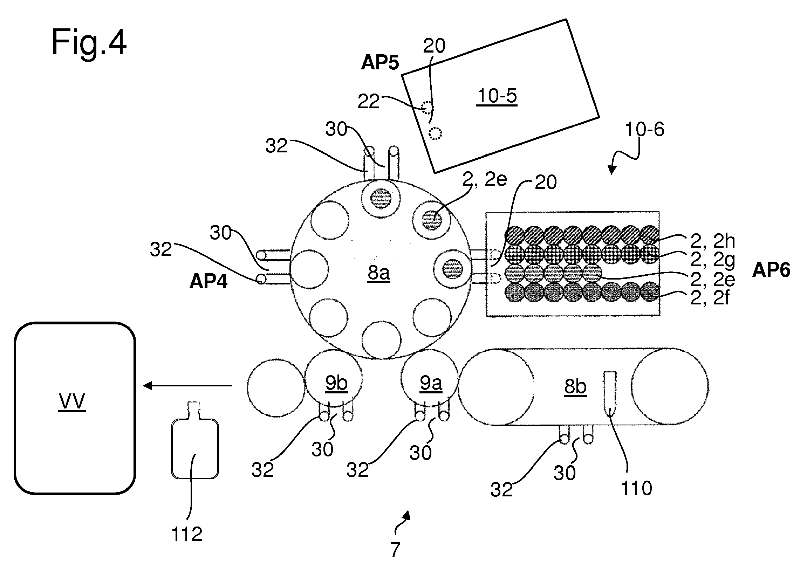

**Figur 4** zeigt die Verwendung mehrerer Wechselautomaten 10 an einer Blasvorrichtung 7. So genannte Vorformlinge 110 werden in einer Heizvorrichtung 8b temperiert und über einen Einlaufstern 9a einem Blasformmodul 8a zugeführt. In dem Blasformmodul 8a werden die Vorformlinge 110 mit Hilfe der richtigen Blasform 2e, 2f, 2g oder 2h in die gewünschte Behälterform 112 gebracht und über einen Auslaufstern 9b weiteren Verarbeitungsvorrichtungen VV zugeführt.

In einer solchen Blasvorrichtung 7 müssen bei einem Produktwechsel eine Reihe von Arbeitswerkzeugen 2 ausgetauscht bzw. Formatteile angepasst werden. In der Heizvorrichtung 8b, einem ggf. nachgeordneten Sterilisationsmodul und / oder einem ggf. nachgeordneten preferential heating Modul müssen beispielsweise Heizdorne zum Transport und zur Erwärmung der Vorformlinge 110 ausgetauscht werden. Des Weiteren finden unterschiedliche Abschirmplatten Verwendung. Diese dienen dem Abschirmen der Mündungsbereiche der Vorformlinge 110 vor der Strahlung während des Transports der Vorformlinge 110 durch die Heizvorrichtung 8b. Auch Transportsternklammern zum Transport der Vorformlinge 110 von der Heizvorrichtung 8b zur Nachbehandlung in einem Sterilisationsmodul oder einem preferential heating Modul oder direkt zum Blasformmodul 8a bzw. zur Entnahme der Flaschen 112 von der Blasform 2e, 2f, 2g oder 2h müssen an das jeweilige Produkt angepasst werden. In der Heizvorrichtung 8b kann weiterhin der Bedarf bestehen, fehlerhafte Infrarotlampen und / oder Reflektoren und / oder ganze Heizkästen mit einer Vielzahl von Lampen und / oder Filter austauschen zu müssen.

Im Blasformmodul 8a selbst müssen beispielsweise die Blasformen 2, 2e gegen andere Blasformen 2f, 2g oder 2h ausgetauscht werden. Insbesondere müssen hierbei die Seitenteile und / oder Einlegeschalen und /oder Böden der Blasformen 2e, 2f, 2g oder 2h ausgetauscht werden. Weitere auszutauschende bzw. anzupassende Arbeitswerkzeuge 2 sind beispielsweise die Reckstangen oder Reckstangenspitzen zum Längsdehnen der Vorformlinge 110 beim Blasen in der Blasform 2e, 2f, 2g oder 2h; die Blasdüsen zum Abdichten der Vorformlinge 110 und zum Zuführen der Blasluft; die Anschläge für die Reckstange - diese dienen zum Stoppen der Reckstange kurz bevor sie den Blasformboden beim Recken erreicht - usw.

Weiterhin kann es in einer solchen Blasvorrichtung 7 notwendig sein, Kontaktelemente zur Temperierung der Vorformlinge 110 vor der Heizvorrichtung 8b oder zwischen Heizvorrichtung 8b und Blasformmodul 8a anzupassen, um insbesondere eine angepasste Temperierung entlang der Umfangsrichtung der Vorformlinge 110 durchführen zu können. Zudem kann ein Austausch von Sterilisationsdüsen, Vorformlings- Führungen und / oder Spritzgussformen notwendig sein. Die Anpassung der Spritzgussformen ist natürlich nur notwendig, wenn der Heizvorrichtung 8b und dem Blasformmodul 8a eine Spritzgusseinrichtung zur Herstellung der Vorformlinge 110 vorgeschaltet ist.

Zum Umrüsten der verschiedenen Module einer Blasvorrichtung, ist an jedem der einzelnen Module - Heizvorrichtung 8b, Blasformmodul 8a, Einlaufstern 9a, Auslaufstern 9b etc. - jeweils mindestens eine Verbindungsvorrichtung 30 mit Zentriervorrichtungen 32 vorgesehen, an die jeweils ein entsprechend bestückter Wechselautomat 10 ankuppeln kann. Der Wechselautomat 10 ist je nach jeweiliger Aufgabe mit den entsprechenden, oben beschriebenen Arbeitswerkzeugen 2 oder notwendigen Umrüst- Werkzeugen zum Anpassen von Formatteilen bestückt. Weiterhin kann vorgesehen sein, dass mehrere unterschiedlich bestückte Wechselautomaten 10 in unterschiedlichen Arbeitspositionen an das Blasformmodul 8a ankuppeln. Beispielsweise kann in der Arbeitsposition AP4 ein mit Reckstangen bestückter Wechselautomat (nicht dargestellt), an der Arbeitsposition AP5 ein mit Reckanschlägen bestückter Wechselautomat 10-5 und an der Arbeitsposition AP6 ein mit Blasformen 2e, 2f, 2g oder 2h bestückter Wechselautomat 10-6 ankoppeln, so dass zeitgleich der Austausch von drei unterschiedlichen Arbeitswerkzeugen 2 durchgeführt werden kann.

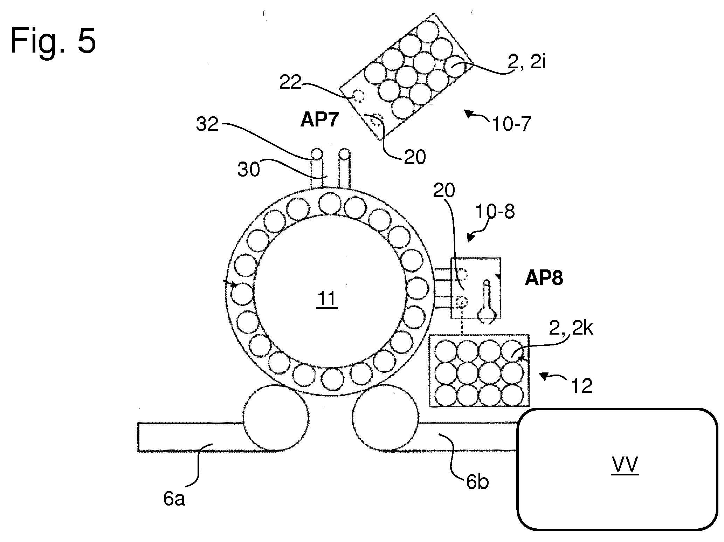

**Figur 5** zeigt die Verwendung mehrerer Wechselautomaten 10 an einem Füller 11. Die zu befüllenden Flaschen werden dem Füller 11 über einen Zuförderer 6a zugeführt und nach dem Befüllen über einen Abförderer 6b beispielsweise einem Verschließer oder einer anderen Verarbeitungsvorrichtungen VV zugeführt. Auch bei einem Füller 11 bzw. bei den einem Füller zugeordneten weiteren Verarbeitungsmodulen, beispielsweise einem Rinser, Sterilisator und / oder Verschließer, müssen verschiedene Arbeitswerkzeuge 2 ausgetauscht oder angepasst werden. Beispielsweise müssen so genannte E-beam-Finger zur Sterilisation der Flaschen mittels Elektronenstrahlung; Sterilisationsdüsen zur Sterilisation der Flaschen mittels chemischer Sterilisationsmittel, beispielsweise mit Wasserstoffperoxid (H₂0₂); Rinsdüsen zum Durchspülen der Flaschen mit Sterilwasser; Düsen der Bodennachkühlung zum Abführen der Wärme aus dem Boden der frisch geblasenen Flaschen und zu deren Verfestigung; Füllventile; Füllrohre zum Abfüllen der Flaschen; Zentrierglocken am Füllventil zum Zentrieren der Flasche während der Abfüllung; Rückluftrohre zum Abführen der Luft aus dem Behälter während der Abfüllung; Verschließkonen oder Verschließköpfe zum Aufbringen / -schrauben / -pressen von Verschlüssen auf die Mündung der Behälter; Einteilschnecken; Führungsschienen für die Flaschen; Dorne, Klammern, Klammergruppen, Halterungen zum Transport von Flaschen etc. an das jeweilige Produkt angepasst werden.

In einer Arbeitsposition AP7 kuppelt beispielsweise ein Wechselautomat 10-7 an, der entsprechend mit Neckhandlingklammern 2i ausgestattet ist, während an der Arbeitsposition AP8 ein Wechselautomat 10-8 für den Austausch der Füllventile 2k vorgesehen ist. Gemäß einer Ausführungsform der Erfindung kann vorgesehen sein, dass der Wechselautomat 10 nicht immer die benötigten Arbeitswerkzeuge 2 selbst bereitstellt, sondern diese einem externen Magazin 12 entnimmt, das dem Füller 11 beispielsweise ortsfest zugeordnet ist.
An dem externen Magazin 12 kann eine ähnliche Verbindung vorhanden sein, wie an den einzelnen Modulen, bzw. die Handhabungsvorrichtung kann zusätzliche Verbindungselemente bzw. Zentrierelemente aufweisen, um an externe Magazine andocken zu können.

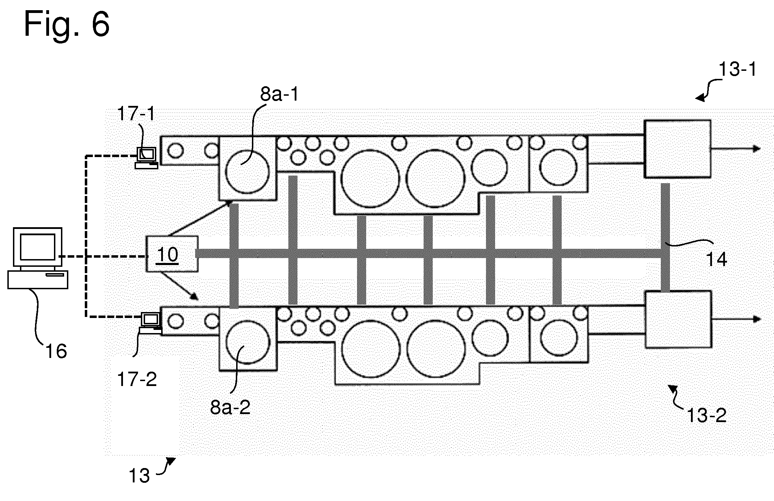

**Figur 6** zeigt die Verwendung eines Wechselautomaten 10 in einer Abfüllanlage 13 mit mindestens zwei Produktionslinien 13-1, 13-2. Die Produktionslinien 13-1, 13-2 fahren vorzugsweise jeweils unterschiedliche Produkte. Somit müssen die beiden Produktionslinien jeweils zu unterschiedlichen Zeitpunkten angepasst werden. Es ist vorgesehen, einen mobilen Wechselautomaten 10 beispielsweise sowohl am Blasformmodul 8a-1 der Produktionslinie 13-1 als auch am Blasformmodul 8a-2 der Produktionslinie 13-2 einsetzen zu können. Weiterhin kann vorgesehen sein, den Wechselautomat 10 nach Austausch der im Magazin angeordneten Arbeitswerkzeuge auch an anderen Modulen der Produktionslinien 13-1, 13-2 einzusetzen. Gemäß einer Ausführungsform dient der Wechselautomat gleichzeitig als Magazin für verschiedenste Arbeitswerkzeuge und / oder Werkzeuge. Alternativ ist vorgesehen, dass jeweils ein Wechselautomat 10 mit Blasformen für die Blasformmodule 8a-1, 8a-2; jeweils ein weiteren Wechselautomat für Heizdorne; jeweils ein weiteren Wechselautomat für Greiferklammern etc. vorgesehen ist. Es sind also für eine Abfüllanlage 13 mit mindestens zwei Produktionslinien 13-1, 13-2 jeweils ein Wechselautomat pro Arbeitswerkzeug 2 vorgesehen, die je nach Bedarf den entsprechenden Austausch bzw. die entsprechende Anpassung an jeweils einer der Produktionslinien 13-1, 13-2 vornehmen.

Die Wechselautomaten 10 sind jeweils fahrbar ausgestaltet, indem sie beispielsweise vom Bediener der Anlage an das jeweilige Modul hingefahren und angekuppelt werden. Alternativ kann vorgesehen sein, dass sich die Wechselautomaten 10 auf einem Schienensystem 14 bewegen, vorzugsweise einen eigenen Antrieb aufweisen und automatisch angesteuert werden. Insbesondere ist die Steuerungsvorrichtung 16 des Wechselautomaten mit den jeweiligen Steuerungsvorrichtungen 17-1 und 17-2 der Produktionslinien 13-1, 13-2 gekoppelt. Die Produktionsdaten der jeweiligen Produktionslinien 13-1, 13-2 sind in der jeweilig zugeordneter Steuerungsvorrichtung 17-1, 17-2 gespeichert. Anhand dieser Daten koordiniert die Steuerungsvorrichtung 16 den Wechselautomaten 10. Insbesondere kann vorgesehen sein, dass die Steuerungsvorrichtung 16 dem Bediener der Anlage rechtzeitig ein Signal gibt, wenn der Wechselautomat 10 neu bzw. mit anderen Arbeitswerkzeugen 2 und / oder Werkzeugen bestückt werden muss. Der Wechselautomat 10 kann auch in der Lage sein, Produktionsaufträge, insbesondere kabellos, zu empfangen und notwendige Umrüstungen selbstständig durchzuführen.

Die Arbeitsposition, an der der Wechselautomat 10 dem jeweiligen Verarbeitungsmodul 8a zugeordnet wird, muss nicht zwingend eine direkte Verbindung zwischen Verarbeitungsmodul 8a und Wechselautomat 10 herstellen. Beispielsweise kann der Wechselautomat 10 auch über korrespondierende Verbindungselemente im Hallenboden einem Verarbeitungsmodul 8a räumlich zugeordnet werden. Es kann auch vorgesehen sein, dass der Wechselautomat 10 über ein eigenes Erkennungssystem verfügt, über das er die gewünschte Position an dem Verarbeitungsmodul 8a erkennt. Dabei kann es sich insbesondere um ein optisches Erkennungssystem handeln. Es kann aber auch eine Erkennung über geeignete elektronische und / oder magnetische Systeme o.ä. erfolgen.

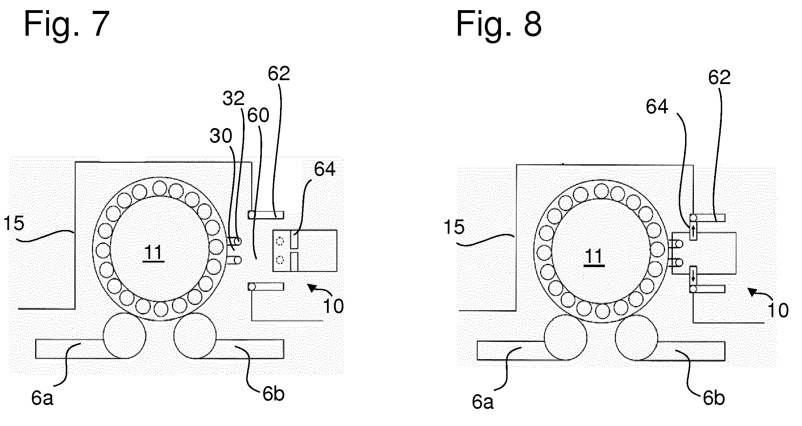

**Figur 7** und **Figur 8** zeigen eine weitere Ausführungsform zur Verwendung eines Wechselautomaten 10 an einem Füller 11. Der Füller 11 ist vorzugsweise von einem Gehäuse 15 umgeben. Das Gehäuse 15 schirmt Schmutz und Verunreinigungen von außen ab. Weiterhin ermöglicht das Gehäuse 15, den Füller steril zu halten. In dem Gehäuse befindet sich mindestens eine durch Sicherheitstüren 62 verschlossene Wechselöffnung 60. Bei einem Produktwechsel werden die Sicherheitstüren 62 geöffnet, so dass ein Wechselautomat 10 mit den entsprechenden Arbeitswerkzeugen und / oder Werkzeugen an die entsprechende Verbindungsvorrichtung 30 ankuppeln kann. Vorzugsweise umfasst der Wechselautomat 10 Sicherheitsvorrichtungen 64, die den Wechselautomaten 64 mit den Sicherheitstüren 62 verriegelt, sobald dieser an den Füller 11 angekoppelt ist. Dadurch wird sichergestellt, dass kein Bediener ungewollt beim Wechselvorgang dazwischen greifen kann.

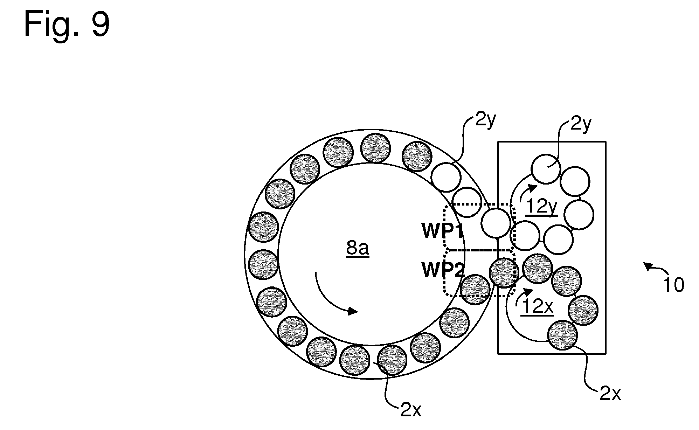

**Figur 9** zeigt eine weitere Ausführungsform eines Wechselautomaten 10, der insbesondere einen kontinuierlichen Austausch von Arbeitswerkzeugen 2, 2x, 2y ermöglicht. Im vorliegenden Fall ist die Verwendung eines solchen Wechselautomaten 10 an einem Blasformmodul 8a dargestellt. Der Wechselautomat 10 umfasst mindestens ein Magazin 12x für die alten Arbeitswerkzeuge 2x, die aus dem Blasformmodul 8a entnommen werden und mindestens ein zweites Magazin 12y für die neuen Arbeitswerkzeuge 2y. Insbesondere ist vorgesehen, dass der Wechselautomat 10 gleichzeitig ein altes Arbeitswerkzeug 2x an einer ersten Wechselposition WP1 entnimmt, während er an eine zweiten, leeren Wechselposition WP2 ein neues Arbeitswerkzeug 2y anbringt. Die Entnahme eines alten Arbeitswerkzeugs 2x und das Befestigen eines neuen Arbeitswerkzeugs 2y erfolgt somit zeitgleich. Eine Ausnahme bildet allerdings die Entnahme des ersten alten Arbeitswerkzeugs 2x. Dies muss dem synchronen Vortausch vorgeschaltet erfolgen, da es nicht möglich ist, ein neues Arbeitswerkzeug 2y an einer Position zu befestigen, die noch durch ein altes Arbeitswerkzeug 2x besetzt ist. Der Austausch erfolgt insbesondere in rotierende Magazine 12x, 12y, in denen der Abstand der Arbeitswerkzeuge 2x, 2y sehr klein gehalten ist, insbesondere kleiner als der Abstand zueinander auf der Behandlungsvorrichtung 8a. Um die Magazine 2x, 2y möglichst klein zu halten, kann deswegen eine spiralförmige Einlagerung / Auslagerung in die Höhe stattfinden. Ebenfalls ist es denkbar, dass der Wechsel durch den Wechselautomaten 10 kontinuierlich abläuft, entweder mit verringerter Geschwindigkeit im Vergleich zur normalen Produktionsgeschwindigkeit der Behandlungsvorrichtung 8a oder im Idealfall sogar mit Produktionsgeschwindigkeit. In beiden Fällen ist daran gedacht, die Produktion während des Wechselns nicht zu unterbrechen. Hierzu kann entweder ein Behälter auf dem Zuförderer oder vorher ausgeschleust werden (oder gar nicht durch die Behandlungsvorrichtung 8a produziert werden), um keinen Behälter in der Behandlung in der entstehenden Lücke nach der Entnahme des ersten Arbeitswerkzeugs 2x zu haben. Theoretisch könnte man auch das Arbeitswerkzeug 2x inklusive dem Behälter entnehmen und der Wechselautomat 10 schleust den Behälter anschließend aus.

Die mit WP1 und WP2 bezeichneten Arbeitsbereiche sind in dieser Ausführungsform weitgehend benachbart an einem einzigen Karussell eines Blasformmoduls angeordnet. Gemäß der in **Figur 9** dargestellten Ausführungsform sind die mit WP1 und WP2 bezeichneten Arbeitsbereiche unmittelbar benachbart an dem Karussell des Blasformmoduls angeordnet und können gleichzeitig durch einen einzigen Wechselautomaten 10 bedient werden.

### Bezugszeichenliste

- 1: Verarbeitungsvorrichtung
- 2: Arbeitswerkzeug bspw. Blasform
- 3: Hebemechanismus
- 4: Übergabevorrichtung
- 5: Monotec- Stern
- 6: Etikettiermodul
- 6a/b: Zuförderer / Abförderer
- 7: Blasvorrichtung
- 8a: Blasformmodul
- 8b: Heizvorrichtung
- 9a/b: Einlaufstern / Auslaufstern
- 10: Wechselautomat
- 11: Füller
- 12: Magazin für Arbeitswerkzeuge und / oder Werkzeuge
- 13: Abfüllanlage
- 13-1/13-2: Produktionslinie
- 14: Schienensystem
- 15: Gehäuse
- 16: Steuerungsvorrichtung
- 17: Steuerungsvorrichtung
- 18: Schiebegriff
- 20: Schnittstelle
- 22: Zentrieröffnung
- 24: elektrische Verbindungselemente
- 30: Verbindungsstelle
- 32: Zentrierstift / Zentrierkonus
- 34: elektrische Verbindungselemente
- 40: Räder
- 44: Sensor
- 46: Anzeigetafel
- 48: Steuerungseinheit
- 50: Batterie
- 52: Antrieb
- 60: Wechselöffnung
- 62: Sicherheitstür
- 64: Sicherheitsvorrichtung
- 100: Etikettieraggregat
- 110: Vorformling
- 112: Behälter / Flasche
- AP: Arbeitsposition
- VV: Verarbeitungsvorrichtung
- WP: Wechselposition

## Patentansprüche

1. Behälterbehandlungsanlage (1, 13) zur Herstellung und / oder Bearbeitung von Produkten und / oder Produktgruppen mit mindestens zwei unterschiedlichen Behälterbehandlungsmodulen, wie z.B. eine Heizstrecke, ein Blasformmodul, ein Etikettiermodul oder ein Füller einer Flaschenherstellungs-, -befüllungs- und/oder -verpackungsaniage, mit mindestens zwei Arbeitsbereichen (AP), wobei die mindestens zwei Arbeitsbereiche (AP) den mindestens zwei unterschiedlichen Behälterbehandlungsmodulen zugeordnet sind, wobei die Arbeitsbereiche (AP) jeweils mindestens ein austauschbares Arbeitswerkzeug (2) und / oder ein anpassbares Formatteil umfassen und wobei die Behälterbehandlungsanlage (1, 13) mindestens eine zu den mindestens zwei Arbeitsbereichen (AP) bewegbare Handhabungsvorrichtung (10) umfasst, welche selektiv mit jeweils einem der Arbeitsbereiche (AP) verbindbar ist und welche Mittel zum Austausch der Arbeitswerkzeuge (2) und / oder zur Anpassung der Formatteile des jeweiligen Arbeitsbereichs (AP) umfasst.

2. Behälterbehandlungsanlage (1, 13) nach Anspruch 1, wobei den Arbeitsbereichen (AP) jeweils mindestens eine erste Verbindungvorrichtung (30) mit Zentriermittel (32) und wobei der Handhabungsvorrichtung (10) jeweils mindestens eine zweite Verbindungsvorrichtung (20) mit Zentriermittel (22) zugeordnet ist, wobei die mindestens eine zweite Verbindungsvorrichtung (20) mit Zentriermittel (22) der Handhabungsvorrichtung (10) korrespondierend zu der jeweiligen ersten Verbindungsvorrichtungen (30) mit Zentriermittel (32) der mindestens zwei Arbeitsbereiche (AP) ausgebildet ist und wobei über die erste Verbindungvorrichtung (30) mit Zentriermittel (32) und die zweite Verbindungsvorrichtung (20) mit Zentriermittel (22) eine form- und / oder kraftschlüssige Verbindung zwischen einem der Arbeitsbereiche (AP) und der Handhabungsvorrichtung (10) herstellbar ist.

3. Behälterbehandlungsanlage (1, 13) nach Anspruch 1 oder 2, wobei die Handhabungsvorrichtung (10) mindestens ein Magazin (12) für Arbeitswerkzeuge (2), und / oder für Werkzeuge umfasst, das mit unterschiedlichen Arbeitswerkzeugen (2) und / oder Werkzeugen bestückbar ist, wobei die Arbeitswerkzeuge (2) aus dem Magazin entnehmbar und austauschbar sind und / oder wobei die Werkzeuge aus dem Magazin entnehmbar und zur Anpassung der Formatteile verwendbar sind.

4. Behälterbehandlungsanlage (1, 13) nach einem der voranstehenden Ansprüche, wobei die Handhabungsvorrichtung (10) Transportmittel (40) umfasst, insbesondere wobei die Handhabungsvorrichtung (10) mit einem eigenen Antrieb (50) zur Bewegung der Handhabungsvorrichtung von einer ersten Arbeitsposition (AP) an der Behälterbehandlungsanlage (1, 13) zu einer zweiten Arbeitsposition (AP) an der Behälterbehandlungsanlage (1, 13) ausgestattet ist.

5. Behälterbehandlungsanlage (1, 13) nach Anspruch 4, wobei die Handhabungsvorrichtung (10) auf Schienen (14) fahrbar angeordnet ist, wobei sich die Schienen zumindest zwischen der ersten Arbeitsposition (AP) und der zweiten Arbeitsposition (AP) an der Behälterbehandlungsanlage (1, 13) erstrecken.

6. Behälterbehandlungsanlage (1, 13) nach einem der voranstehenden Ansprüche, wobei die Handhabungsvorrichtung (10) eine Steuerungsvorrichtung (48) umfasst, wobei die Steuerungsvorrichtung den Austausch der Arbeitswerkzeuge an der jeweiligen Arbeitsposition (AP) und / oder die Anpassung der Formatteile an der jeweiligen Arbeitsposition (AP) und / oder die korrekte Positionierung der Handhabungsvorrichtung (10) an der jeweiligen Arbeitsposition (AP) steuert.

7. Behälterbehandlungsanlage (1, 13) nach einem der Ansprüche 1 bis 5, wobei die Behälterbehandlungsanlage (1, 13) oder mindestens ein Behälterbehandlungsmodul (5, 8, 11) eine Steuerung (16, 17) umfasst und wobei die Handhabungsvorrichtung (10) an die Steuerung (16, 17) der Behälterbehandlungsanlage (1, 13) oder des Behälterbehandlungsmoduls (5, 8, 11) koppelbar ist.

8. Behälterbehandlungsanlage (1, 13) nach einem der voranstehenden Ansprüche, wobei die Handhabungsvorrichtung (10) und die Arbeitsbereiche (AP) der Behälterbehandlungsanlage (1, 13) Schnittstellen für die Versorgung der Handhabungsvorrichtung mit Energie (32), Druckluft und / oder Information aufweist.

9. Behälterbehandlungsanlage (1, 13) nach einem der Ansprüche 2 bis 8, wobei die Handhabungsvorrichtung (10) sensorische Mittel (44) zur Erkennung von Arbeitswerkzeugen (2) und / oder Werkzeugen umfasst, insbesondere wobei die Handhabungsvorrichtung (10) einen Sensor (44) zur Erkennung fehlerhafter Arbeitswerkzeuge (2) und / oder Werkzeuge umfasst.

10. Behälterbehandlungsanlage (1, 13) nach einem der voranstehenden Ansprüche, wobei die Handhabungsvorrichtung (10) mindestens ein Leer- Magazin (12x) für die Aufnahme der entnommenen Arbeitswerkzeuge (2x) aufweist und mindestens ein mit anzubringenden Arbeitswerkzeuge (2y) befüllbares Speicher- Magazin (12y) umfasst und wobei die Entnahme der alten Arbeitswerkzeuge (2x) und das Anbringen der neuen Arbeitswerkzeuge (2y) durch die Handhabungsvorrichtung (10) simultan durchführbar ist.

11. Handhabungsvorrichtung (10) zum Austauschen von Arbeitswerkzeugen (2) und / oder zur Anpassung von Formatteilen an mindestens zwei unterschiedlichen Arbeitsbereichen (AP) einer Behälterbehandlungsanlage (1, 13) nach einem der Ansprüche 1 bis 10, wobei die Handhabungsvorrichtung (10) Mittel zum Austausch der unterschiedlichen Arbeitswerkzeuge (2) und/oder zur Anpassung der unterschiedlichen Formatteile der jeweiligen Arbeitsbereiche (AP), und mindestens eine zweite Verbindungsvorrichtung (20) mit Zentriermittel (22) umfasst, wobei den mindestens zwei Arbeitsbereichen (AP) an mindestens zwei unterschiedlichen Behälterbehandlungsmodulen der Behälterbehandlungsanlage (1, 13) jeweils eine erste Verbindungvorrichtung (30) mit Zentriermittel (32) zugeordnet ist, wobei die mindestens eine zweite Verbindungsvorrichtung (20) mit Zentriermittel (22) der Handhabungsvorrichtung (10) korrespondierend zu den ersten Verbindungsvorrichtungen (30) mit Zentriermittel (32) der mindestens zwei Arbeitsbereiche (AP) ausgebildet ist und wobei eine form- und / oder kraftschlüssige Verbindung zwischen jeweils einem der Arbeitsbereiche (AP) und der Handhabungsvorrichtung (10) herstellbar ist und wobei die Handhabungsvorrichtung (10) mobil an jeweils einem der mindestens zwei Arbeitsbereiche (AP) einsetzbar ist.

12. Handhabungsvorrichtung (10) nach Anspruch 11, wobei die die Handhabungsvorrichtung (10) mindestens ein Leer- Magazin (12x) für die Aufnahme der entnommenen Arbeitswerkzeuge (2x) aufweist und mindestens ein mit anzubringenden Arbeitswerkzeugen (2y) befüllbares Speicher- Magazin (12y) umfasst und wobei die Entnahme der alten Arbeitswerkzeuge (2x) und das Anbringen der neuen Arbeitswerkzeuge (2y) durch die Handhabungsvorrichtung (10) simultan durchführbar ist.

## Claims

1. Container treatment system (1, 13) for producing and / or processing products and / or groups of products with at least two different container processing modules, e.g. a heating alley, a blow molding module, a labelling module or a filler of a bottle production, bottle filling and / or bottle packaging plant, comprising at least two working areas (AP), wherein the at least two working areas (AP) are assigned to the at least two different container processing modules, wherein the working areas (AP) each comprise at least one exchangeable working tool (2) and / or one adaptable format part and wherein the container treatment system (1, 13) comprises at least one handling device (10) that is movable to the at least two working areas (AP), which handling device can be selectively connected, in each case, to one of the working areas (AP), and which handling device comprises means for exchanging the working tools (2) and / or for adapting the format parts of the respective working area (AP).

2. Container treatment system (1, 13) according to claim 1, wherein the working areas (AP) are respectively assigned at least one first connection device (30) having centering means (32) and wherein the handling device (10) is respectively assigned at least one second connection device (20) having centering means (22), wherein the at least one second connection device (20) with centering means (22) of the handling device (10) is formed correspondingly to the respective first connection devices (30) with centering means (32) of the at least two working areas (AP) and wherein the first connection device (30) having centering means (32) and the second connection device (20) having centering means (22) together may serve to create a form- and / or force- locking connection between one of the working areas (AP) and the handling device (10).

3. Container treatment system (1, 13) according to claim 1 or 2, wherein the handling device (10) comprises at least one magazine (12) for working tools (2), and / or for tools, which magazine may be filled with different working tools (2) and / or different tools, wherein the working tools (2) may be removed from the magazine and exchanged and / or wherein the tools may be retrieved from the magazine and used for adapting the format parts.

4. Container treatment system (1, 13) according to one of the previous claims, wherein the handling device (10) comprises transport means (40), in particular wherein the handling device (10) is equipped with its own drive (50) for moving the handling device from a first working position (AP) at the container treatment device (1, 13) to a second working position (AP) at the container treatment device (1, 13).

5. Container treatment system (1, 13) according to claim 4, wherein the handling device (10) is arranged to be moveable along tracks (14) and wherein the tracks extend at least between the first working position (AP) and the second working position (AP) at the container treatment system (1, 13).

6. Container treatment system (1, 13) according to one of the previous claims, wherein the handling device (10) comprises a control device (48), wherein the control device controls the exchange of the working tools at the respective working position (AP) and/ or the adaptation of the format parts at the respective working position (AP) and / or the correct positioning of the handling device (10) at the respective working position (AP).

7. Container treatment system (1, 13) according to one of the claims 1 to 5, wherein the container treatment system (1, 13) or at least one container treatment module (5, 8, 11) comprises a control device (16, 17) and wherein the handling device (10) may be coupled to the control device (16, 17) of the container treatment system (1, 13) or of the container treatment module (5, 8, 11).

8. Container treatment system (1, 13) according to one of the previous claims, wherein the handling device (10) and the working areas (AP) of the container treatment system (1, 13) comprise interfaces for supplying power (32), compressed air, and / or data to the handling device.

9. Container treatment system (1, 13) according to one of the claims 2 to 8, wherein the handling device (10) comprises sensory means (44) for detecting working tools (2) and / or tools, in particular wherein the handling device (10) comprises a sensor (44) for detecting defective working tools (2) and / or defective tools.

10. Container treatment system (1, 13) according to one of the previous claims, wherein the handling device (10) comprises at least one empty magazine (12x) for receiving the removed working tools (2x) and at least one storage magazine (12y), into which the working tools (2y) for being mounted can be filled and wherein the handling device (10) may perform the removal of the former working tools (2x) and the mounting of the new working tools (2y) simultaneously.

11. Handling device (10) for exchanging working tools (2) and / or for adapting format parts of at least two different working areas (AP) of a container treatment system (1, 13) according to one of the claims 1 to 10, wherein the handling device (10) comprises meais for exchanging different working tools (2) and / or for adapting differend format parts of the respective working areas (AP) and wherein the handling device comprises at least one second connection device (20) with centering means (22) and wherein one first connection device (30) with centering means (32) is assigned to each of the at least two different working areas (AP) of at least two different container processing modules of the container treatment system (1, 13), wherein the at least one second connection device (20) with centering means (22) of the handling device (10) is formed correspondingly to the first connection device (30) with centering means (32) of the at least two working areas (AP) and wherein a form- and / or force locking connection can be created in each case between one of the working areas (AP) and the handling device (10), and wherein the handling device (10) is movable and usable respectively at each of the at least two working areas (AP).

12. Handling device (10) according to claim 11, wherein the handling device (10) comprises at least one empty magazine (12x) for receiving the removed working tools (2x) and at least one storage magazine (12y), into which the working tools (2y) for being mounted can be filled and wherein the handling device (10) may perform the removal of the former working tools (2x) and the mounting of the new working tools (2y) simultaneously.

## Revendications

1. Installation de traitement de récipients (1, 13) pour la fabrication et/ou le traitement de produits et/ou de groupes de produits, comprenant au moins deux modules différents de traitement de récipients, tels que, par exemple, une section de chauffage, un module de moulage par soufflage, un module d'étiquetage ou une machine à remplir d'une installation de fabrication de bouteilles, de remplissage de bouteilles et/ou de conditionnement de bouteilles, ayant au moins deux zones de travail (AP), dans laquelle lesdites au moins deux zones de travail (AP) sont associées auxdits au moins deux modules différents de traitement de récipients, dans laquelle lesdites zones de travail (AP) comprennent chacune au moins un outil de travail (2) échangeable et/ou une pièce de format adaptable et l'installation de traitement de récipients (1, 13) comprend au moins un dispositif de manipulation (10) déplaçable vers lesdites au moins deux zones de travail (AP) qui peut être relié sélectivement à l'une des zones de travail (AP) respectivement et qui comprend des moyens pour échanger les outils de travail (2) et/ou pour adapter les pièces de format de la zone de travail (AP) respective.

2. Installation de traitement de récipients (1, 13) selon la revendication 1, dans laquelle respectivement au moins un premier dispositif de liaison (30) à moyen de centrage (32) est associé aux zones de travail (AP) et dans laquelle respectivement au moins un deuxième dispositif de liaison (20) à moyen de centrage (22) est associé au dispositif de manipulation (10), ledit au moins un deuxième dispositif de liaison (20) à moyen de centrage (22) du dispositif de manipulation (10) étant réalisé de manière correspondante au premier dispositif de liaison (30) respectif à moyen de centrage (32) desdites au moins deux zones de travail (AP), et dans laquelle une liaison à engagement positif et/ou par adhérence peut être réalisée entre l'une des zones de travail (AP) et le dispositif de manipulation (10) par le premier dispositif de liaison (30) à moyen de centrage (32) et par le deuxième dispositif de liaison (20) à moyen de centrage (22).

3. Installation de traitement de récipients (1, 13) selon la revendication 1 ou 2, dans laquelle le dispositif de manipulation (10) comprend au moins un magasin (12) pour des outils de travail (2) et/ou pour des outils qui peut être équipé de différents outils de travail (2) et/ou outils, dans laquelle les outils de travail (2) peuvent être retirés du magasin et être échangés et/ou dans laquelle les outils peuvent être retirés du magasin et être utilisés pour adapter les pièces de format.

4. Installation de traitement de récipients (1, 13) selon l'une quelconque des revendications précédentes, dans laquelle le dispositif de manipulation (10) comprend des moyens de transport (40), dans laquelle, en particulier, le dispositif de manipulation (10) est équipé d'un propre mécanisme d'entraînement (50) pour déplacer le dispositif de manipulation depuis une première position de travail (AP) sur l'installation de traitement de récipients (1, 13) vers une deuxième position de travail (AP) sur l'installation de traitement de récipients (1, 13).

5. Installation de traitement de récipients (1, 13) selon la revendication 4, dans laquelle ledit dispositif de manipulation (10) est disposé de manière mobile sur rails (14), lesdits rails s'étendant au moins entre la première position de travail (AP) et la deuxième position de travail (AP) sur l'installation de traitement de récipients (1, 13).

6. Installation de traitement de récipients (1, 13) selon l'une quelconque des revendications précédentes, dans laquelle le dispositif de manipulation (10) comprend un dispositif de commande (48), ledit dispositif de commande commandant l'échange des outils de travail sur la position de travail (AP) respective et/ou l'adaptation des pièces de format sur la position de travail (AP) respective et/ou le positionnement correct du dispositif de manipulation (10) sur la position de travail (AP) respective.

7. Installation de traitement de récipients (1, 13) selon l'une quelconque des revendications 1 à 5, dans laquelle l'installation de traitement de récipients (1, 13) ou au moins un module de traitement de récipients (5, 8, 11) comprend une commande (16, 17) et dans laquelle le dispositif de manipulation (10) peut être accouplé à la commande (16, 17) de l'installation de traitement de récipients (1, 13) ou du module de traitement de récipients (5, 8, 11).

8. Installation de traitement de récipients (1, 13) selon l'une quelconque des revendications précédentes, dans laquelle le dispositif de manipulation (10) et les zones de travail (AP) de l'installation de traitement de récipients (1, 13) présentent des interfaces pour alimenter ledit dispositif de manipulation en énergie (32), en air comprimé et/ou en information.

9. Installation de traitement de récipients (1, 13) selon l'une quelconque des revendications 2 à 8, dans laquelle le dispositif de manipulation (10) comprend des moyens détecteurs (44) destinés à détecter des outils de travail (2) et/ou des outils, dans laquelle, en particulier, le dispositif de manipulation (10) comprend un capteur (44) destiné à détecter des outils de travail (2) et/ou outils défectueux.

10. Installation de traitement de récipients (1, 13) selon l'une quelconque des revendications précédentes, dans laquelle le dispositif de manipulation (10) présente au moins un magasin vide (12x) pour recevoir les outils de travail (2x) retirés et comprend au moins un magasin de stockage (12y) apte être rempli d'outils de travail (2y) à monter, et dans laquelle les actions de retirer les vieux outils de travail (2x) et de monter les nouveaux outils de travail (2y) peuvent être effectuées simultanément par le dispositif de manipulation (10).

11. Dispositif de manipulation (10) destiné à échanger des outils de travail (2) et/ou à adapter des pièces de format sur au moins deux zones de travail (AP) différentes d'une installation de traitement de récipients (1, 13) selon l'une quelconque des revendications 1 à 10, dans lequel le dispositif de manipulation (10) comprend des moyens d'échange des outils de travail (2) différents et/ou d'adaptation des pièces de format différentes des zones de travail (AP) respectives ainsi qu'au moins un deuxième dispositif de liaison (20) à moyen de centrage (22), dans lequel respectivement un premier dispositif de liaison (30) à moyen de centrage (32) est associé auxdites au moins deux zones de travail (AP) sur au moins deux modules différents de traitement de récipients de l'installation de traitement de récipients (1, 13), ledit au moins un deuxième dispositif de liaison (20) à moyen de centrage (22) du dispositif de manipulation (10) étant réalisé de manière correspondante aux premiers dispositifs de liaison (30) à moyen de centrage (32) desdites au moins deux zones de travail (AP), et une liaison à engagement positif et/ou par adhérence pouvant être réalisée entre respectivement l'une des zones de travail (AP) et le dispositif de manipulation (10), et ledit dispositif de manipulation (10) pouvant être utilisé de manière mobile sur respectivement l'une desdites au moins deux zones de travail (AP).

12. Dispositif de manipulation (10) selon la revendication 11, dans lequel le dispositif de manipulation (10) présente au moins un magasin vide (12x) pour recevoir les outils de travail (2x) retirés et comprend au moins un magasin de stockage (12y) apte être rempli d'outils de travail (2y) à monter, et dans lequel les actions de retirer les vieux outils de travail (2x) et de monter les nouveaux outils de travail (2y) peuvent être effectuées simultanément par le dispositif de manipulation (10).
